# EUROPÄISCHE PATENTSCHRIFT

(11) **EP 2 512 589 B1**
(45) Veröffentlichungstag und Bekanntmachung des Hinweises auf die Patenterteilung: **01.06.2016**
(21) Anmeldenummer: 10771008.9
(22) Anmeldetag: 13.10.2010
(51) Int. Cl.: A61N 1/32

(54) **VORRICHTUNG ZUM VERABREICHEN VON ARZNEIMITTELN UND ZUM BEEINFLUSSEN VON ARZNEIMITTELWIRKUNGEN**
DEVICE FOR ADMINISTERING DRUGS AND FOR INFLUENCING THE EFFECTS OF DRUGS
DISPOSITIF PERMETTANT D'ADMINISTRER DES MÉDICAMENTS ET D'INFLUENCER LES EFFETS DE MÉDICAMENTS

(30) Priorität: 13.10.2009 DE 102009049175
(43) Veröffentlichungstag der Anmeldung: 24.10.2012
(73) Patentinhaber: Geiges, Bjarne, 80538 München (DE)
(72) Erfinder: Geiges, Bjarne, 80538 München (DE)
(74) Vertreter: Schumacher & Willsau
(86) Internationale Anmeldenummer: PCT/EP2010/006271
(87) Internationale Veröffentlichungsnummer: WO 2011/045053

(56) Entgegenhaltungen:
- WO-A1-2004/098697
- WO-A2-2008/007369
- US-A1- 2004 267 169

## Beschreibung

Die Erfindung betrifft eine Vorrichtung zum Verabreichen von Arzneimitteln und zum Beeinflussen von Arzneimittelwirkungen.

Eine Vorrichtung zum Verabreichen von Arzneimitteln in einem Blutgefäß wird in der WO 2004/098697 A1 beschrieben.

Im Zusammenhang mit der Verabreichung von Arzneimitteln besteht das grundsätzliche Problem, dass diese in der Regel ohne oder mit nur wenig lokalisierenden Maßnahmen appliziert werden. Es ist also nicht nur ein erkrankter Körperbereich, der mit den Wirkstoffen in Kontakt kommt, sondern vielmehr werden auch gesunde Körperzellen durch die verabreichten Substanzen beeinträchtigt und unter Umständen nachhaltig geschädigt.

Ein weiteres Problem, das die Wirksamkeit von Arzneimitteln beeinträchtigt, hängt damit zusammen, dass die zu penetrierenden Zellmembranen und die biochemischen Schutzhüllen von Zellen ein Hindernis für die Arzneimittel darstellen. Beispielsweise können hierdurch Bakterien gegen Antibiotika resistent werden, und Krebszellen können trotz der Anwendung von Zytostatika überleben. Ebenfalls kann es problematisch sein, wachstumsfördernde Substanzen oder sonstige zelluläre Wirkstoffe so zu applizieren, dass die Zellmembranen penetriert werden.

Aber auch wenn die wirksame Substanz das Innere der Zelle erreicht hat, kann es intrazelluläre Reaktionshemmnisse geben, die vielfach schlicht mit der Kinetik der erforderlichen chemischen Reaktion zusammenhängen, also insbesondere mit dem Fehlen der für die Reaktion erforderlichen Aktivierungsenergie.

Der Erfindung liegt die Aufgabe zugrunde, den genannten Problemen einzeln oder insgesamt entgegenzutreten, insbesondere also Arzneimittel lokal zu konzentrieren, die Penetration von Zellmembranen zu fördern und die Kinetik der intrazellulären Reaktion zu beeinflussen.

Diese Aufgabe wird mit den Merkmalen des unabhängigen Anspruches gelöst.

Vorteilhafte Ausführungsformen der Erfindung sind in den abhängigen Ansprüchen angegeben.

Die Erfindung betrifft eine Vorrichtung nach Anspruch 1. Eine solche Vorrichtung lässt sich in der direkten Umgebung des erkrankten Körperbereiches platzieren. Durch die Spannungsquelle beziehungsweise das elektromagnetische Feld, welches sich in der Umgebung des elektrischen Leiters ausbildet, kann die Freisetzung der von dem Substrat aufgenommenen Arzneimittel bewirkt beziehungsweise gefördert werden. Der vorliegende verwendete Begriff "Substrat" kann spezieller auch durch den Begriff "Matrix" ersetzt werden. Körperregionen, die sich entfernt von der lokalisiert platzierten Vorrichtung befinden, werden durch die lokal applizierten Arzneimittel nicht oder nur wenig erreicht. Ferner sorgt die Anwesenheit eines elektrischen Feldgradienten dafür, dass die Penetration der Arzneimittel durch die Zellmembranen erleichtert wird. Ebenfalls kann das elektrische Feld für eine Konzentration an ionischen Botenstoffen (z.B. Ca²⁺) sorgen, so dass auch hierdurch die Penetration durch die Membranporenöffnung gefördert wird.

Es ist besonders bevorzugt, dass eine Einrichtung zum Beeinflussen einer durch die Spannungsquelle erzeugten elektrischen Spannung vorgesehen ist. Hierdurch kann die Abgabe von Arzneimitteln aus dem Substrat gesteuert werden. Die Vorrichtung kann zum Beispiel so ausgelegt sein, dass bei einer Erhöhung der elektrischen Spannung eine erhöhte Arzneimittelabgabe stattfindet.

Es ist vorgesehen, dass der mit dem elektrischen Pol verbundene elektrische Leiter ein Metalldraht ist. Grundsätzlich können jegliche elektrische Leiter verwendet werden. Ein Metalldraht, insbesondere ein Edelstahldraht, stellt jedoch eine nützliche Möglichkeit dar, die von der Spannungsquelle erzeugten elektrischen Potentiale zu übertragen.

Nützlicherweise ist dabei vorgesehen, dass der Metalldraht das Substrat trägt. Neben den für die Erfindung erforderlichen elektrischen Eigenschaften des Metalldrahtes stellt dieser auch eine sichere und flexible Möglichkeit zur Verfügung, das Arzneimittel enthaltende Substrat aufzunehmen. Das Substrat kann aus einer unlöslichen Matrix wie PMMA (Polymethylmetaccylat) und in Form von beispielsweise Kugeln oder Ellipsoiden von einem Träger, in der Regel ein Stahldraht, ähnlich einer Perlenkette aufgenommen werden, die im Bereich einer Infektion, beispielsweise einer Knochen-Infektion implantiert wird.

Im Zusammenhang mit der vorliegenden Erfindung ist es besonders nützlich, dass die Spannungsquelle einen Übertrager umfasst, der bei Anwesenheit eines externen Magnetfeldes eine elektrische Spannung erzeugt. Ein externes magnetisches Wechselfeld kann in einem Übertrager, der insbesondere durch eine Spule realisiert ist, eine elektrische Spannung erzeugen. Die Variation des externen Magnetfeldes kann mithin auch eine Variation der elektrischen Spannung und somit der Abgaberate des Arzneimittels bewirken. Fernen kann durch das magnetische Wechselfeld die Reaktionskinetik in der Weise beeinflusst werden, dass die Reaktion der Zellsubstanzen mit dem Arzneimittel bereits bei niedrigerer Aktivierungsenergie ablaufen kann.

Ebenfalls kann vorgesehen sein, dass die Spannungsquelle eine Batterie umfasst. Eine solche Batterie kann insbesondere zusätzlich zu einem Übertrager vorgesehen sein, der ein elektrisches Wechselfeld erzeugt, nämlich zum Überlagern der Wechselspannung durch eine Gleichspannungskomponente.

Die vorliegende Erfindung ist in besonders nützlicher Weise dadurch weitergebildet, dass ein zumindest teilweise elektrisch leitfähiges Implantat elektrisch mit einem Pol der Spannungsquelle gekoppelt ist. Gerade im Bereich von Endoprothesen und Osteosynthesemitteln kann die Erfindung in vorteilhafter Weise zum Einsatz kommen. Zum Einen ist es bekannt, dass die Anwesenheit von niederfrequenten magnetischen Wechselfeldern und die hierdurch mittels Übertrager bewirkte elektrische Stimulation von Implantaten einen positiven Einfluss auf die Förderung und die Differenzierung des Gewebewachstums hat. Beispielhafte Systeme sind in den folgenden Dokumenten beschrieben: DE 199 28 449 C1, DE 10 2004 024 473 A1, DE 10 2006 018 191 B4, DE 10 2007 049 542 A1 und DE 10 2007 063 027 A1. Bei den hier dargestellten Vorrichtungen und Verfahren werden in Implantaten elektrische Wechselpotentiale niedriger Frequenz dadurch erzeugt, dass ein betroffener Körperteil einem magnetischen Wechselfeld ausgesetzt wird. Seit langem wurde in zahlreichen klinischen Anwendungen dieser Technik bei chronisch therapieresistenten, meist infizierten Knochendefekten, Zysten und Tumormetastasen sowie in kliniknahen experimentellen Studien gezeigt, dass mit der Verwendung von Implantaten als Quellen extrem niederfrequenter sinusförmiger elektrischer Wechselpotentiale in der dem Stützmetall anliegenden Knochenregion ein optimaler Heilungseffekt erzielt wird. Vergleichbares gilt bei Endoprothesen. Die Technik der Übertragung funktioniert nach dem Transformatorprinzip: Die verletzte oder kranke Körperregion wird von einem extrem niederfrequenten sinusförmig verlaufenden Magnetfeld mit einer Frequenz von ca. 1 bis 100 Hz - vorzugsweise von 4 bis 20 Hz - und einer magnetischen Flussdichte von 0,5 bis 5 mT (5 bis 50 Gauß) durchflutet, das durch einen Funktionsstromgenerator in einer oder mehreren - primären - äußeren Stromspulen erzeugt wird, in die das mit den Implantaten versehene Körperteil eingebracht wird. Diese extrem niederfrequenten elektromagnetischen Felder durchdringen weitgehend verlustfrei das Gewebe, einschließlich eventuelle Kleidung und einen Gipsverband, sowie die unmagnetischen (austenitischen) Stützmetalle der Implantate. Im elektrischen Kontakt mit diesen wird eine - sekundäre - Spulenanordnung, der so genannte Übertrager, implantiert. Die in dem Übertrager induzierten Elektropotentiale werden so im Bereich der Knochenläsion sowie allgemein in dem an die Implantate angrenzenden Gewebe zur Wirkung gebracht. Mit der Erfindung wird nun erreicht, dass einerseits die Implantate in der beschriebenen Art und Weise elektrifiziert werden. Andererseits wird mit den selben Mitteln eine lokale Verabreichung von Arzneimitteln ermöglicht.

Gemäß einer speziellen Ausführungsform der Erfindung ist vorgesehen, dass eine Zahn-Kiefer-Brücke vorgesehen ist, die zumindest teilweise das Substrat bildet oder das Substrat trägt und die weiterhin die Spannungsquelle sowie die mit der Spannungsquelle zusammenwirkenden Elektroden enthält. Eine solche Zahn-Kiefer-Brücke kann durch ihre knochenbildende Wirkung die komplizierte mechanische Callusdistraktion des alveolaren Kieferbereiches ersetzen. Weiterhin können infizierte Zahnprothesen in Formae der enossalen Kieferimplantate - die sogenannte Periimplantitis - erfolgreich behandelt werden. Die Elektroden der Zahn-Kiefer-Brücke sind dabei vorzugsweise buccal und lingual angeordnet. Es sind Gleich- oder Wechselspannungen im Bereich von 500 bis 700 mV zwischen den Elektroden erreichbar, mit denen das Knochenwachstum stimuliert und eine stark antibiotische Wirkung entwickelt wird. Bakterien-Biofilme werden von ihrem Träger, beispielsweise einer Zahnprothese aus einer Titanlegierung, gelöst, und ihre Virulenz wird beendet, aufgrund derer zur Zeit noch circa 20 bis 30 Prozent aller Zahnprothesen verloren gehen. Neben der Behandlung der Periimplantitis kann dieser durch die spezielle Ausführungsform der Erfindung mit Zahn-Kiefer-Brücke aber auch vorgebeugt werden. An der Zahnfehlstelle im Kiefer wird durch die Zahn-Kiefer-Brücke und die hierdurch ermöglichte Elektroosteotherapie das Knochenwachstum stimuliert, so dass der Kiefer für eine spätere Implantation vorbereitet wird. Gleichzeitig wird durch die antibakterielle Wirkung der Zahn-Kiefer-Brücke eine Infektion im Vorfeld der Implantation ausgeschlossen.

In diesem Zusammenhang ist es besonders nützlich, dass die Zahn-Kiefer-Brücke zwei Brückenpfosten und ein Brückenplateau enthält, dass in dem Brückenplateau die Spannungsquelle angeordnet ist und dass in den Brückenpfosten jeweils eine mit je einem Pol der Spannungsquelle verbundene Elektrode sowie das Substrat im Berührungskontakt mit Gingiva und Mucosa angeordnet sind.

Gemäß einer bevorzugten Ausführungsform der Vorrichtung zur Kieferbehandlung ist vorgesehen, dass mindestens eine Komponente, welche das Substrat und die mit der Spannungsquelle zusammenwirkende Elektrode trägt, mittels eines plastischen Abdrucks eines zu kontaktierenden Körperteils gefertigt ist. Aufgrund der stark welligen und profilierten Oberflächen des Zahn- und Kieferbereiches ist es problematisch, mit herkömmlichen Gerätschaften einen ausreichenden Kontakt zwischen den Elektroden und den zu kontaktierenden Körperteilen herzustellen. Beispielsweise würde eine Stützplatte, wie sie bei der Osteosynthese von Frakturen langer Röhrenknochen zur Anwendung kommt, aufgrund mangelnden elektrischen Kontaktes an stimulierender Wirksamkeit verlieren. Auch im Hinblick auf die Freisetzung von Arzneimitteln wäre eine einfache Platte nicht optimal, denn die Freisetzung hängt von der Größe der Oberfläche des Substrats im Verhältnis zu Ausdehnung des Defektbereiches ab. Diese Problematiken werden durch die Verwendung eines plastischen Abdruckes gelöst. Vorzugsweise werden sowohl buccal als auch lingual Kunststoffabdrücke gefertigt, auf die dann letztlich eine elektrisch leitfähige, poröse und kompressible Schicht aufgebracht wird. Diese Schicht bildet das Substrat für das Arzneimittel. Es ist möglich, vor dem Aufbringen der elektrisch leitfähigen, porösen und kompressiblen Schicht eine leitende Oberflächenschicht auf die Abdrücke aufzubringen, die insbesondere zur Kontaktierung der von der Spannungsquelle herangeführten Anschlüsse dient. Die plastischen Abdrücke werden vorzugsweise von einer Klammer gehalten, die auch als Träger der Aufnehmerspule und/oder der Batterie und/oder eines Gleichstrom-/Wechselstromgenerators dienen können. Die elektrisch leitfähigen, porösen und kompressiblen Schichten können ganz oder teilweise aus einem elektrisch leitfähigen Kunststoff bestehen, beispielsweise aus Polymeren, welche durch Dotierung und/oder Beschichtung in eine elektrisch leitfähige Form überführt werden können.

Ebenfalls kann vorgesehen sein, dass mindestens eine Komponente, welche das Substrat und die mit der Spannungsquelle zusammenwirkende Elektrode bildet, durch ein zumindest teileweis leitfähiges und saugfähiges Material gebildet ist. Die Fertigung plastischer Abdrücke erfordert einen gewissen Aufwand. Dieser kann vermieden werden, indem die Elektroden insgesamt aus einem schwammartigen elastischen Material mit guter elektrischer Leitfähigkeit gefertigt werden. Es kommen dieselben Materialien in Betracht, wie für die elektrisch leitfähige, poröse und kompressible Beschichtung der plastischen Abdrücke. Während bei den plastischen Abdrücken die elektrisch leitfähigen Strukturen vorzugsweise ausschließlich dort aufgebracht werden, wo ein elektrischer Kontakt zum Körper hergestellt werden soll und wo ebenfalls die Arzneimittel abgegeben werden sollen, ist es bei den insgesamt aus porösem Material bestehenden Elektroden sinnvoll, die buccalen und lingualen Bereiche elektrisch zu isolieren und auch den Austritt von Arzneimitteln an diesen Stellen zu verhindert, indem die Oberflächen dort in eine flüssigkeitsundurchlässige Form, beispielsweise durch Beschichtung, überführt werden.

Gemäß einer bevorzugten Ausführungsform der Erfindung ist vorgesehen, dass das Substrat zumindest teilweise aus Polymethylmetacrylat besteht. Polymethylmetacrylat (PMMA) ist ein biologisch verträglicher Wirkstoff, der zudem geeignet ist, Arzneimittel aufzunehmen und am Ort der Erkrankung wieder abzugeben.

Es ist besonders bevorzugt, dass das Substrat zumindest teilweise aus einer Magnesium-Kalzium-Legierung oder Magnesium-Zink-Kalzium-Legierung besteht. Ein Nachteil der Verwendung von PMMA zeigt sich beim explantieren aus dem Gewebe, in das die Substratkette mehr oder weniger eingewachsen ist, so dass und zu ihrer Entnahme ein größerer chirurgischer Eingriff erforderlich ist. Dieser Nachteil kann erfindungsgemäß durch die Verwendung von Substraten vermieden werden, die aus biokompatiblem, im Gewebe löslichen Material bestehen. Ein besonders geeignetes Substrat dafür bildet Magnesium in Legierungen mit Kalzium und/oder Zink und/oder auch mit Lithium und/oder seltenen Erden. Wegen seiner unmittelbaren Verwandtschaft zur Kalziumphosphat, dem Hauptbestandteil der harten Knochensubstanz, eignet sich die Legierung von Magnesium mit Kalzium besonders für die antibiotische Behandlung infizierter Knochenareale. Eine Magnesiumlegierung kann auch als Träger von Knochenwachstumsfaktoren dienen, beispielsweise BMP ("bone morphogenetic protein") oder TGF-Beta ("transforming growth factor beta"). Einen weiteren Vorteil von Magnesium als Arzneimittelträger bietet in der erfindungsgemäßen Anordnung dessen elektrisches Leitvermögen, das mit 22,6 S/m zwischen Aluminium (36,6 S/m) und Messing mit (15,5 S/m) rangiert.

Die Erfindung besteht weiterhin in einem System mit einer erfindungsgemäßen Vorrichtung zum Anbringen an einem oder Einbringen in einen menschlichen Körper und mit einer Einrichtung zum Erzeugen eines körperexternen Magnetfeldes.

Weiterhin betrifft die Erfindung ein Verfahren zum Herstellen einer erfindungsgemäßen Vorrichtung, wobei im Rahmen der Herstellung der Vorrichtung mindestens eine Komponente, welche das Substrat und die mit der Spannungsquelle zusammenwirkende Elektrode trägt, mittels eines plastischen Abdruckes eines zu kontaktierenden Körperteils gefertigt wird.

Die Erfindung wurde vorstehend hauptsächlich im Zusammenhang mit Implantaten beschreiben. Hierauf ist sie jedoch nicht beschränkt. Eine weitere nützliche Anwendung ist beispielsweise ein Wundverband mit Elektrodennetz und einem Antibiotikum, insbesondere Gentamyzin, wobei ein Kollagengeflecht das Substrat bildet.

Der Erfindung liegt die Erkenntnis zugrunde, dass durch eine einheitliche Technologie gleichzeitig die Prinzipien der Elektro-Osteotherapie angewendet werden können und ferner den eingangs geschilderten Problemkreisen im Zusammenhang mit der Applizierung von Arzneimitteln begegnet werden kann. Arzneimittel können lokal verabreicht werden, die Penetration von Zellmembranen wird erleichtert und die Kinetik der intrazellulären Reaktion wird beschleunigt. Die Anwesenheit von Magnetfeldern kann noch auf andere Weise genutzt werden, indem nämlich die Arzneistoffe an einen paramagnetischen Träger gekoppelt werden. Dabei enthält der Träger Nanopartikel, die die erforderlichen magnetischen Eigenschaften aufweisen. Indem der Bereich des Patienten, an dem die Wirkstoffe konzentriert werden sollen, nun so in einem räumlich veränderlichen Magnetfeld, einem sogenannten Gradientenmagnetfeld, angeordnet wird, dass sich die Substanzen im Bereich der zu therapierenden Zellen, beispielsweise im Bereich eines Tumors, konzentrieren, können andere Körperregionen von den zu verabreichenden Wirkstoffen unbeeinflusst bleiben. Die Wirkstoffe werden also lokal in der Nähe der zu behandelnden Körperregionen appliziert, und sie konzentrieren sich dann aufgrund der magnetischen Gradientenfelder im betroffenen Gewebe. Dieses Verfahren wird auch als "Magnetofektion" bezeichnet.

Die Erfindung wird nun mit Bezug auf die begleitenden Zeichnungen anhand besonders bevorzugter Ausführungsformen beispielhaft erläutert.

Es zeigen:
- Figur 1: eine Ausführungsform einer erfindungsgemäßen Vorrichtung;
- Figur 2: eine weitere Ausführungsform einer erfindungsgemäßen Vorrichtung;
- Figur 3: eine an ein Implantat gekoppelte Ausführungsform der vorliegenden Erfindung;
- Figur 4: eine an ein weiteres Implantat gekoppelte Ausführungsform der vorliegenden Erfindung;
- Figur 5: eine weitere Ausführungsform der vorliegenden Erfindung;
- Figur 6: eine als Zahn-Kiefer-Brücke ausgebildete Ausführungsform der vorliegenden Erfindung;
- Figur 7: eine Seitenansicht der Ausführungsform gemäß Figur 6;
- Figur 8: eine weitere Seitenansicht der Ausführungsform gemäß Figur 6;
- Figur 9: eine Einzelheit der Ausführungsform gemäß Figur 6;
- Figur 10: eine Ausführungsform eines Zahnspacers mit Elektrode;
- Figur 11: eine weitere Ausführungsform eines Zahnspacers mit Elektrode;
- Figur 12: eine weitere Ausführungsform eines Zahnspacers mit Elektrode;
- Figur 13: eine spezielle Ausführungsform einer erfindungsgemäßen Vorrichtung zur Verwendung im Kieferbereich und
- Figur 14: eine weitere Ausführungsform einer erfindungsgemäßen Vorrichtung zur Verwendung im Kieferbereich.

Bei der nachfolgenden Beschreibung der Zeichnungen bezeichnen gleiche Bezugszeichen gleiche oder vergleichbare Komponenten.

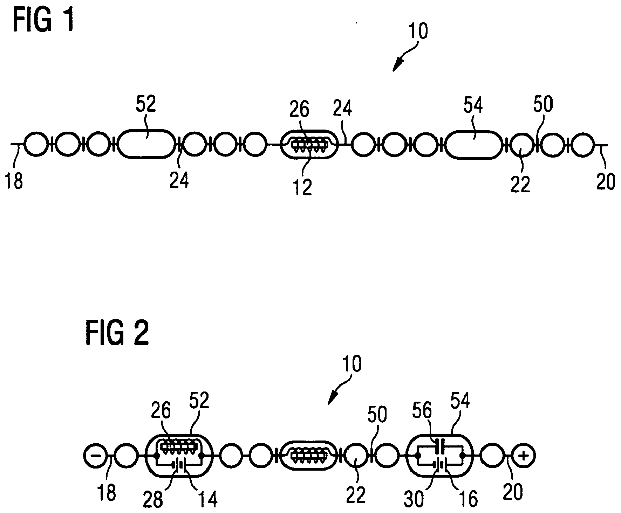

Figur 1 zeigt eine Ausführungsform einer erfindungsgemäßen Vorrichtung. Die Vorrichtung 10 weist eine Spannungsquelle 12 auf, die hier als Übertrager 26 realisiert ist. Der Übertrager 26 kann eine Spulenwicklung auf einem weichmagnetischen Kern sein. An der Spannungsquelle 12 schließen an den jeweiligen Polen Metalldrähte 24 an, die als Elektroden 18, 20 wirken. Die Metalldrähte 24 tragen Kugeln, die ein Substrat 22 für Arzneimittel bilden. Das Substrat 22 kann auch durch andersartig geformte Elemente gebildet sein. Die einzelnen Elemente des Substrats 22 sind durch optional vorzusehende Abstandshalter 50 aus leitendem oder nichtleitendem Material, das heißt Abstandshalter in Form von Plättchen aus dem gleichen Material, des die Substrat-Kugeln (Elypsoide) tragenden Drahtes, voneinander getrennt. Auf den Metalldrähten 24 befinden sich Aufnahmeinrichtungen 52, 54, in denen weitere Komponenten angeordnet werden können, beispielsweise weitere Spannungsquellen oder sonstige elektrische Elemente.

Befindet sich die Vorrichtung 10 gemäß Figur 1 in einem magnetischen Wechselfeld, so liegt zwischen den Elektroden 18, 20 eine Wechselspannung an. Aufgrund des elektromagnetischen Feldes kann die Abgabe des Arzneimittels aus dem Substrat 22 gefördert werden. Durch Variation des äußeren Magnetfeldes und mithin des elektrischen Feldes kann die Arzneimittelabgabe in ihrer Geschwindigkeit gesteuert werden. Als Substrat kommt insbesondere Polymethylmetacrylat (PMMA) in Frage. Ebenfalls können resorbierbare Träger verwendet werden. Beispielsweise können für Antibiotika Collagen, Calciumsulfat und Magnesium zum Einsatz kommen.

Figur 2 zeigt eine weitere Ausführungsform einer erfindungsgemäßen Vorrichtung. Die hier dargestellte Anordnung ist vergleichbar zu derjenigen aus Figur 1. Zusätzlich sind elektrische Bauteile zu erkennen, die innerhalb der Aufnahmeeinrichtungen 52, 54 angeordnet sind. So befindet sich innerhalb der Aufnahmeeinrichtungen 52 eine Batterie 28, die der von dem externen Wechselmagnetfeld erzeugten Wechselspannung einen Gleichspannungsanteil aufprägt. Der Batterie 28 kann, wie im vorliegenden Beispiel dargestellt, ein weiterer Übertrager 26 parallel geschaltet sein. Die Aufnahmeeinrichtung 54 enthält ebenfalls eine Batterie 30. Dieser ist im vorliegenden Beispiel ein Kondensator 56 parallel geschaltet. Durch die Kombination einer eine Gleichspannung erzeugenden Batterie mit dem elektrischen Wechselfeld, welches durch ein externes Magnetfeld stimuliert wird, kann die Wechselspannung durch direkte Einflussnahme auf das externe Magnetfeld variiert werden, und gleichzeitig können die Elektroden 18, 20 umgebende Gewebe als Anode und Kathode festgelegt werden, was einen Einfluss auf die Abgabe des Arzneimittels und auf das die Elektroden 18, 20 nehmen kann. Die Parallelschaltung eines Kondensators 56 zu einer Gleichspannungsquelle 30 erlaubt die Applikation einer Gleichspannung mit überlagerter Wechselspannungskomponente, deren Amplitude von der Größe des Kondensators abhängt. Als Kondensatoren können beispielsweise Elektrolytkondensatoren oder Supercaps verwendet werden. Ebenfalls ist es möglich, die Vorrichtung mit reiner Gleichspannung zu betreiben, also entweder mit einer gleichgerichteten von einem Übertrager erzeugten Spannung oder alleine auf der Grundlage einer Batterie. Die Anordnung wird nur bei vorgeschaltetem Übertrager wirksam, der auf die Drahtelektrode ein elektrisches Wechselpotential überträgt, das durch den parallel zur Batterie geschalteten Kondensator diese überbrückt.

Beispielhafte Betriebsarten können wie folgt charakterisiert werden:
- Es wird eine reine elektromagnetisch induzierte Wechselspannung mit einer Frequenz zwischen 10 und 20 Hz verwendet.
- Es wird eine reine Gleichspannung aus einer integrierten Batterie von 700 bis 800 mV verwendet.
- Durch die Kombination einer Gleichspannungsquelle und einer Wechselspannungsquelle wird eine Dauergleichspannung in der Höhe von 200 bis 400 mV erzeugt und von einer Wechselspannung mit einer Amplitude von ca. 500 mV temporär oder dauerhaft überlagert.

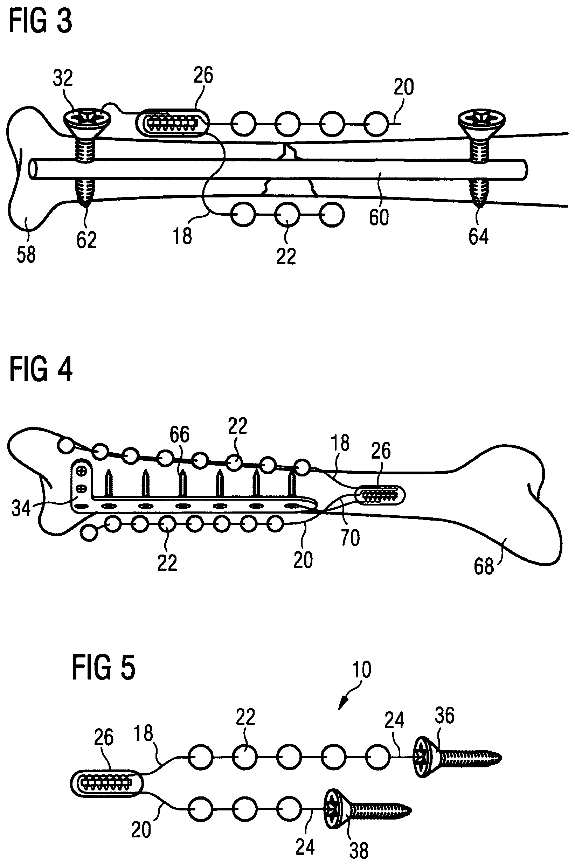

Figur 3 zeigt eine an ein Implantat gekoppelte Ausführungsform der vorliegenden Erfindung. Ein Knochen 58 ist mit einem Marknagel 60 ausgestattet. Der Marknagel 60 wird durch Verriegelungsschrauben 62, 64 fixiert. Diese Implantate, im vorliegenden Beispiel insbesondere das Implantat 32, nämlich eine der Verriegelungsschrauben, steht mit einem Pol eines Übertragers 26 in Verbindung. An dem Übertrager 26 sind weiterhin Elektroden 18 angeordnet, die die im Zusammenhang mit den Figuren 1 und 2 erwähnten Arzneimittelsubstrate 22 tragen. Dabei kann die Ankopplung an den Übertrager 26 so erfolgen, dass die Elektroden 18, 20 auf dem selben Potential oder auf entgegengesetzten Potentialen liegen, je nach Bedarf. Durch die dargestellte Anordnung gelingt es also, einerseits das Implantat 32 im Rahmen der Elektro-Osteotherapie einzusetzen und die vom Übertrager 26 erzeugte elektrische Spannung gleichzeitig im Hinblick auf die Freisetzung von Arzneimitteln zu benutzen.

Figur 4 zeigt eine an ein weiteres Implantat gekoppelte Ausführungsform der vorliegenden Erfindung. Das hier dargestellte Implantat 34 ist eine Knochenstützplatte, die durch eine Mehrzahl von Schrauben 66 an einem Knochen 68 fixiert ist. Eine Spannungsquelle, hier insbesondere wieder ein Übertrager 26, ist einerseits über eine elektrische Leitung 70 mit der Knochenstützplatte verbunden. Weiterhin schließen zwei Elektroden 18, 20 an, die Substrate 22 mit Arzneimitteln tragen. Wiederum kann der elektrische Anschluss der Komponenten am Übertrager 26 auf verschiedene Arten realisiert sein. Beispielsweise kann die Knochenstützplatte auf einem entgegengesetzten Potential bezüglich der beiden Elektroden 18, 20 liegen. Ebenfalls ist es denkbar, die Knochenstützplatte mit einem Mittelabgriff der Spule im Übertrager 26 zu verbinden, während die beiden Elektroden 18, 20 die beiden entgegengesetzt geladenen Elektroden bilden. Auch kann die Knochenstützplatte auf dem selben Potential wie eine der beiden entgegengesetzt gepolten Elektroden 18, 20 liegen.

Figur 5 zeigt eine weitere Ausführungsform der vorliegenden Erfindung. Bei der hier gezeigten Vorrichtung 10 ist der Übertrager 26 über die Elektroden 18, 20 mit jeweils einer Schraube 36, 38 verbunden. Die Metalldrähte 24, die zu den Schrauben 36, 38 führen, tragen wiederum das Substrat 22. Hier bilden die Schrauben 36, 38 die Elektroden 18, 20 mit entgegengesetztem Potential. Als Schrauben 36, 38 können beispielsweise solche verwendet werden, die mit Titan-Niob-Oxinitrid beschichtet sind.

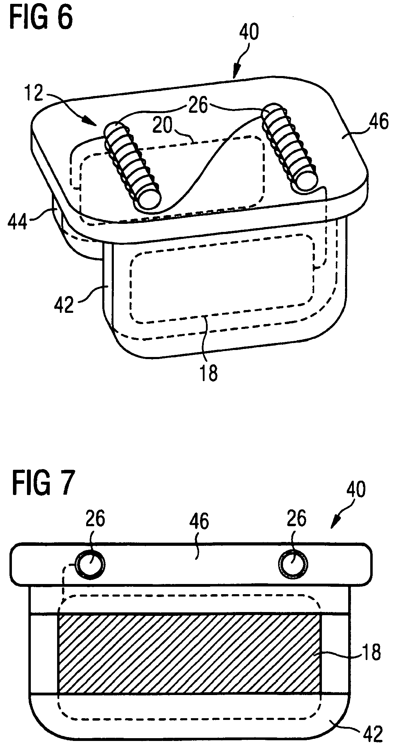

Figur 6 zeigt eine als Zahn-Kiefer-Brücke ausgebildete Ausführungsform der vorliegenden Erfindung. Die hier dargestellte Zahn-Kiefer-Brücke 40 dient der Formung des alveolaven Kieferknochens. Die Zahn-Kiefer-Brücke 40 umfasst zwei Brückenpfosten 42, 44 und ein Brückenplateau 46. In dem Brückenplateau 46 ist die Spannungsquelle 12 angeordnet, hier wiederum beispielhaft ein Übertrager 26, der zwei elektrisch hintereinander geschaltete Spulen umfasst. In den Brückenpfosten 42, 44 sind die Elektroden 18, 20 angeordnet, wobei die Elektrode 18 im Brückenpfosten 42 und die Elektrode 20 im Brückenpfosten 44 liegt. Die Induktionsspulen des Übertragers 46 sind im vorliegenden Beispiel über flache Eisenkerne gewickelt und mit Teflon oder Epoxydharz ummantelt. Vergleichbar können die Elektroden 18, 20 ganz oder teilweise ummantelt sein.

Figur 7 zeigt eine Seitenansicht der Ausführungsform gemäß Figur 6. Es ist einer der Brückenpfosten 42 mit darin liegender Elektrode 18 erkennbar. Ferner ist das Brückenplateau 46 mit dem darin eingebetteten Übertrager 26 zu erkennen.

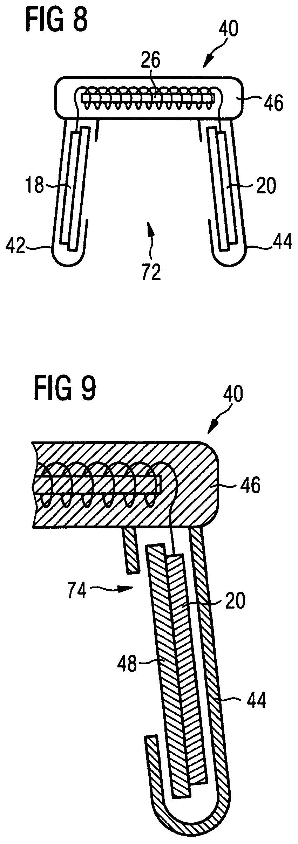

Figur 8 zeigt eine weitere Seitenansicht der Ausführungsform gemäß Figur 6. In dieser Darstellung erkennt man beide Brückenpfosten 42, 44 mit den jeweiligen Elektroden 18, 20 sowie das Brückenplateau 46 mit dem Übertrager 26. Es ist auch der Bereich 72 zu erkennen, in den der Kieferknochen einwachsen soll.

Figur 9 zeigt eine Einzelheit der Ausführungsform gemäß Figur 6. Hier ist zusätzlich zur Elektrode 20 in dem dargestellten Brückenpfosten 44 ein Substrat 48 erkennbar, welches benachbart zur Elektrode 20 angeordnet ist und für welches auf der dem Hohlraum zugewandten Seite des Brückenpfostens 44 eine Öffnung 74 vorgesehen ist, über die das vom Substrat 48 aufgenommene Arzneimittel in den Hohlraum 74 abgegeben werden kann.

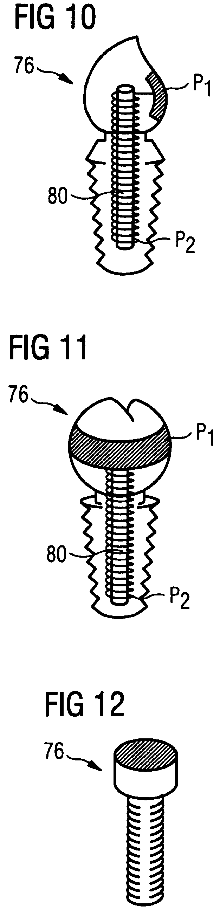

Figur 10 zeigt eine Ausführungsform eines Zahnspacers ("tooth spacer") mit Elektrode. Hier ist ein Beispiel für einen Zahnspacer 76 zum Einschrauben in das Gewinde eines im Kieferknochen implantierten Trägers einer Zahnprothese gezeigt. Der Zahnspacer 76 enthält in seinem Inneren einen Übertrager 80, der zwei verschiedene elektrisch gegeneinander isolierte Bereiche des Zahnspacers 76 auf entgegengesetztes Potential P₁ und P₂ bringt. Der Übertrager 80 wird zur Stimulation des Knochenwachstums durch das selbe externe magnetische Wechselfeld zur Erzeugung einer elektrischen Spannung angeregt, welches auch den Übertrager in der Zahn-Kiefer-Brücke anregt, durch den letztlich die Arzneimittelabgabe aus dem Substrat 48 (siehe Figur 9) stimuliert beziehungsweise gesteuert werden kann.

Die Figuren 11 und 12 zeigen zwei weitere konstruktive Beispiele für Zahnspacer 76 mit Elektrode, wobei in der Figur 12 wieder der interne Übertrager 80 erkennbar ist.

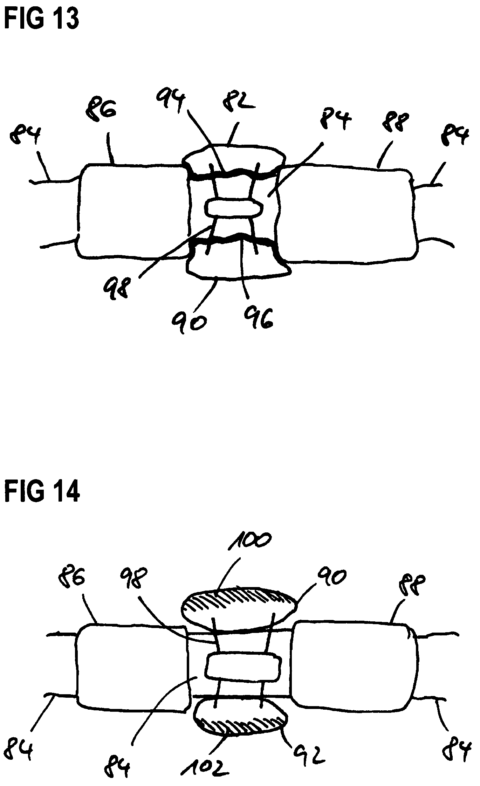

Figur 13 zeigt eine spezielle Ausführungsform einer erfindungsgemäßen Vorrichtung zur Verwendung im Kieferbereich. Es ist ein Teil eines Kiefers 84 mit zwei intakten Zähnen 86, 88 dargestellt. Zwei seitlich des Kiefers lingual und buccal angeordnete Komponenten 82, 90 liegen passgenau an dem Kiefer 84 an. Dies ist erreichbar, indem die Komponenten 82, 90 mittels eines plastischen Abdrucks des zu kontaktierenden Kiefers 84 gefertigt werden. Nach Herstellung des plastischen Abdruckes wird letztlich eine poröse, elektrisch leitfähige und kompressible Schicht 94, 96 auf die durch plastischen Abdruck gefertigten Komponenten 82, 90 aufgebracht, wobei diese Schichten 94, 96 insbesondere den Bereich der Komponenten 82, 90 abdecken sollen, die dem Kiefer 84 zugewandt sind. Die dem Kiefer abgewandten Seiten der Komponenten 82, 90 sollen keine elektrische Leitfähigkeit haben und auch keine Aufnahmekapazität für Arzneimittel aufweisen, was bei plastischen Abdrücken in der Regel von vornherein gewährleistet ist. Vor dem Aufbringen der kompressiblen, elektrisch leitfähigen und porösen Schichten 94, 96 auf die Komponenten 82, 90, kann zunächst eine herkömmliche elektrische Beschichtung auf die Komponenten 82, 90 aufgebracht werden, die dann die elektrisch leitfähigen, porösen und kompressiblen Schichten trägt. Diese Zwischenbeschichtung dient in erster Linie der Kontaktierung der Spannungsquelle. Die Komponenten 82, 90 werden dann von einer Klammer 98 gehalten. Die Klammer trägt auch den Übertrager und/oder Batterie und/oder den Gleichstrom-/Wechselstromgenerator, und sie führt die elektrischen Spannungen den mit Elektroden beschichteten Komponenten 82, 90 zu. Im vorliegenden Fall, bei dem eine Zahnlücke zwischen zwei intakten Zähnen 86, 88 vorliegt, ist ausreichend Raum zwischen den Zähnen, um die Klammer 98 zusammen mit den Komponenten 82, 96 anzuordnen. Soll eine erfindungsgemäße Vorrichtung eingesetzt werden, um eine bereits bestehende Periimplantitis bei einem bereits implantierten Implantat zu heilen, so wird die Klammer in der Regel auf dem Implantat nach Art einer Beißschiene aufsitzen.

Figur 14 zeigt eine weitere Ausführungsform einer erfindungsgemäßen Vorrichtung zur Verwendung im Kieferbereich. Die hier dargestellte Vorrichtung ähnelt in weiten Teilen derjenigen gemäß Figur 13. Die Komponenten 90, 92 gemäß Figur 14 unterscheiden sich jedoch von den Komponenten 82, 90 gemäß Figur 13. Die Komponenten 90, 92 gemäß Figur 14 werden nämlich insgesamt durch ein poröses, elektrisch leitfähiges und kompressibles beziehungsweise elastisches Material gebildet. Die schraffierten Bereiche 100, 102 sind durch beispielsweise Beschichtungen elektrisch isoliert und flüssigkeitsundurchlässig gestaltet. Durch die Wirkung der Klammer 98 schmiegen sich die insbesondere als Ellipsoide gestalteten Komponenten 90, 92 dem Kiefer 84 an, und sie sorgen so für die gute elektrische Kontaktierung, welche für den Erfolg der das Knochenwachstum stimulierenden antibiotischen Behandlung erforderlich ist.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

### Bezugszeichenliste

- 10: Vorrichtung
- 12: Spannungsquelle
- 14: Spannungsquelle
- 16: Spannungsquelle
- 18: Elektroden
- 20: Elektroden
- 22: Substrat
- 24: Metalldrähte
- 26: Übertrager
- 28: Batterie
- 30: Batterie
- 32: Implantat
- 34: Implantat
- 36: Schraube
- 38: Schraube
- 40: Zahn-Kiefer-Brücke
- 42: Brückenpfosten
- 44: Brückenpfosten
- 46: Brückenplateau
- 48: Substrat
- 50: Abstandshalter
- 52: Aufnahmeinrichtung
- 54: Aufnahmeinrichtung
- 56: Kondensator
- 58: Knochen
- 60: Marknagel
- 62: Verriegelungsschraube
- 64: Verriegelungsschraube
- 66: Schrauben
- 68: Knochen
- 70: elektrische Leitung
- 72: Hohlraum
- 74: Öffnung
- 76: Zahnspacer
- 80: Übertrager
- 82: Komponente
- 84: Kiefer
- 86: Zahn
- 88: Zahn
- 90: Komponente
- 92: Komponente
- 94: Schicht
- 96: Schicht
- 98: Klammer
- 100: isolierter Bereich
- 102: isolierter Bereich

## Patentansprüche

1. Vorrichtung (10) mit
- einer Spannungsquelle (12, 14, 16),
- einem mit einem elektrischen Pol der Spannungsquelle verbundenen als Elektrode (18, 20) wirkenden elektrischen Leiter und
- einem im elektromagnetischen Feld, das sich in der Umgebung des elektrischen Leiters bei aktiver Spannungsquelle (12, 14, 16) ausbildet, angeordneten Substrat (22), wobei das Substrat ein darin aufgenommenes Arzneimittel enthält,
- wobei eine Einrichtung zum Beeinflussen einer durch die Spannungsquelle (12, 14, 16) erzeugten elektrischen Spannung vorgesehen ist,
- wobei der mit dem elektrischen Pol verbundene elektrische Leiter ein Metalldraht (24) ist,
- wobei der Metalldraht (24) das Substrat (22, 48) trägt und
- wobei die Spannungsquelle (12) einen Übertrager (26) umfasst, der bei Anwesenheit eines externen magnetischen Wechselfeldes eine elektrische Wechselspannung im Metalldraht erzeugt, so dass eine Variation des externen magnetischen Wechselfeldes eine Variation der elektrischen Wechselspannung und somit der Abgaberate des Arzneimittels bewirkt.

2. Vorrichtung (10) nach Anspruch 1, **dadurch gekennzeichnet, dass** die Spannungsquelle (14, 16) eine Batterie (28, 30) umfasst.

3. Vorrichtung (10) nach Anspruch 1 oder 2, **dadurch gekennzeichnet, dass** ein zumindest teilweise elektrisch leitfähiges Implantat (32, 34, 36, 38) elektrisch mit einem Pol der Spannungsquelle (12) gekoppelt ist.

4. Vorrichtung (10) nach Anspruch 1, **dadurch gekennzeichnet, dass** eine Zahn-Kiefer-Brücke (40) vorgesehen ist, die zumindest teilweise das Substrat bildet oder das Substrat trägt und die weiterhin die Spannungsquelle sowie die mit der Spannungsquelle zusammenwirkende Elektrode enthält.

5. Vorrichtung (10) nach Anspruch 4, **dadurch gekennzeichnet, dass** die Zahn-Kiefer-Brücke (40) zwei Brückenpfosten (42, 44) und ein Brückenplateau (46) enthält, dass in dem Brückenplateau (46) die Spannungsquelle angeordnet ist und dass in den Brückenpfosten jeweils eine mit je einem Pol der Spannungsquelle verbundene Elektrode sowie das Substrat (48) angeordnet sind.

6. Vorrichtung nach Anspruch 4, **dadurch gekennzeichnet, dass** mindestens eine Komponente (82, 90), welche das Substrat (22) und die mit der Spannungsquelle zusammenwirkende Elektrode (18, 20) trägt, mittels eines plastischen Abdrucks eines zu kontaktierenden Körperteils (84) gefertigt ist.

7. Vorrichtung nach Anspruch 4, **dadurch gekennzeichnet, dass** mindestens eine Komponente (90, 92), welche das Substrat und die mit der Spannungsquelle zusammenwirkende Elektrode (18, 20) bildet, durch ein zumindest teileweis leitfähiges und saugfähiges Material gebildet ist.

8. Vorrichtung (10) nach einem der vorangehenden Ansprüche, **dadurch gekennzeichnet, dass** das Substrat (48) zumindest teilweise aus Polymethylmetacrylat besteht.

9. Vorrichtung (10) nach einem der vorangehenden Ansprüche, **dadurch gekennzeichnet, dass** das Substrat (48) zumindest teilweise aus einer Magnesium-Kalzium-Legierung oder Magnesium-Zink-Kalzium-Legierung besteht.

10. System mit einer Vorrichtung (10) nach einem der vorangehenden Ansprüche zum Anbringen an einem oder Einbringen in einen menschlichen Körper und mit einer Einrichtung zum Erzeugen eines körperexternen Magnetfeldes.

11. Verfahren zum Herstellen einer Vorrichtung (10) nach einem der Ansprüche 1 bis 9 wobei im Rahmen der Herstellung der Vorrichtung mindestens eine Komponente (82), welche das Substrat (22) und die mit der Spannungsquelle zusammenwirkende Elektrode (18, 20) trägt, mittels eines plastischen Abdrucks eines zu kontaktierenden Körperteils (84) gefertigt wird.

## Claims

1. Device (10) comprising
- a voltage source (12, 14, 16),
- an electrical conductor connected to an electrical pole of the voltage source and acting as an electrode (18, 20); and
- a substrate (22) arranged in the electromagnetic field that forms in the vicinity of the electrical conductor when the voltage source (12, 14, 16) is active, the substrate containing a drug held therein,
- wherein a device for influencing an electrical voltage generated by the voltage source (12, 14, 16) is provided,
- wherein the electrical conductor connected to the electrical pole is a metal wire (24),
- wherein the metal wire (24) carries the substrate (22, 48) and
- wherein the voltage source (12) comprises a transformer (26), which generates an alternating electrical voltage in the metal wire when an external alternating magnetic field is applied, so that a variation of the external alternating magnetic field brings about a variation of the alternating electrical voltage and consequently of the dispensing rate of the drug.

2. Device (10) according to Claim 1, **characterized in that** the voltage source (14, 16) comprises a battery (28, 30).

3. Device (10) according to Claim 1 or 2, **characterized in that** an at least partially electrically conductive implant (32, 34, 36, 38) is electrically coupled to a pole of the voltage source (12).

4. Device (10) according to Claim 1, **characterized in that** a tooth-jaw bridge (40) is provided, at least partially forming the substrate or carrying the substrate and also containing the voltage source as well as the electrode interacting with the voltage source.

5. Device (10) according to Claim 4, **characterized in that** the tooth-jaw bridge (40) comprises two bridge abutments (42, 44) and a bridge platform (46), **in that** the voltage source is arranged in the bridge platform (46) and **in that** an electrode connected to a respective pole of the voltage source and the substrate (48) are respectively arranged in the bridge abutments.

6. Device according to Claim 4, **characterized in that** at least one component (82, 90) that carries the substrate (22) and the electrode (18, 20) interacting with the voltage source is produced by means of a plastic impression of a body part (84) to be contacted.

7. Device according to Claim 4, **characterized in that** at least one component (90, 92) that forms the substrate and the electrode (18, 20) interacting with the voltage source is formed by at least partially conductive and absorbent material.

8. Device (10) according to one of the preceding claims, **characterized in that** the substrate (48) at least partially consists of polymethyl methacrylate.

9. Device (10) according to one of the preceding claims, **characterized in that** the substrate (48) at least partially consists of a magnesium-calcium alloy or a magnesiumzinc-calcium alloy.

10. System comprising a device (10) according to one of the preceding claims for attaching to or introducing into the human body and with a device for generating a magnetic field outside the body.

11. Method for producing a device (10) according to one of Claims 1 to 9, wherein, in the course of the production of the device, at least one component (82) that carries the substrate (22) and the electrode (18, 20) interacting with the voltage source is produced by means of a plastic impression of a body part (84) to be contacted.

## Revendications

1. Dispositif (10) comprenant
- une source de tension (12, 14, 16),
- un conducteur électrique relié à un pôle électrique de la source de tension et faisant office d'électrode (18, 20) et
- un substrat (22) disposé dans le champ électromagnétique qui se forme dans l'environnement du conducteur électrique lorsque la source de tension (12, 14, 16) est activée, le substrat contenant un médicament absorbé dans celui-ci,
- un appareil destiné à influencer une tension électrique générée par la source de tension (12, 14, 16) étant présent,
- le conducteur électrique relié au pôle électrique étant un fil métallique (24),
- le fil métallique (24) portant le substrat (22, 48) et
- la source de tension (12) comprenant une bobine translatrice (26) qui, en présence d'un champ magnétique alternatif externe, génère une tension électrique alternative dans le fil métallique de sorte qu'une variation du champ magnétique alternatif externe provoque une variation de la tension électrique alternative et ainsi du taux de diffusion du médicament.

2. Dispositif (10) selon la revendication 1, **caractérisé en ce que** la source de tension (14, 16) comprend une batterie (28, 30).

3. Dispositif (10) selon la revendication 1 ou 2, **caractérisé en ce qu'**un implant (32, 34, 36, 38) au moins partiellement conducteur d'électricité est connecté électriquement à un pôle de la source de tension (12).

4. Dispositif (10) selon la revendication 1, **caractérisé en ce qu'**il existe un pont dent-mâchoire (40) qui forme au moins partiellement le substrat ou qui porte le substrat et qui contient en outre la source de tension ainsi que l'électrode qui coopère avec la source de tension.

5. Dispositif (10) selon la revendication 4, **caractérisé en ce que** le pont dent-mâchoire (40) contient deux piliers de pont (42, 44) et un plateau de pont (46), **en ce que** la source de tension est disposée dans le plateau de pont (46) et **en ce qu'**une électrode reliée avec respectivement un pôle de la source de tension ainsi que le substrat (48) sont respectivement disposés dans les piliers de pont.

6. Dispositif selon la revendication 4, **caractérisé en ce qu'**au moins un composant (82, 90), qui porte le substrat (22) ainsi que l'électrode (18, 20) qui coopère avec la source de tension, est fabriqué au moyen d'une empreinte en plastique d'une partie du corps (84) avec laquelle un contact doit être établi.

7. Dispositif selon la revendication 4, **caractérisé en ce qu'**au moins un composant (90, 92), qui forme le substrat ainsi que l'électrode (18, 20) qui coopère avec la source de tension, est constitué d'un matériau au moins partiellement conducteur et absorbant.

8. Dispositif (10) selon l'une des revendications précédentes, **caractérisé en ce que** le substrat (48) est constitué au moins partiellement de polyméthacrylate de méthyle.

9. Dispositif (10) selon l'une des revendications précédentes, **caractérisé en ce que** le substrat (48) est constitué au moins partiellement d'un alliage de magnésium et de calcium ou d'un alliage magnésium-zinc-calcium.

10. Système comprenant un dispositif (10) selon l'une des revendications précédentes destiné à être apposé sur ou incorporé dans un corps humain et comprenant un appareil destiné à générer un champ magnétique extracorporel.

11. Procédé de fabrication d'un dispositif (10) selon l'une des revendications 1 à 9, au moins un composant (82), qui porte le substrat (22) ainsi que l'électrode (18, 20) qui coopère avec la source de tension, étant fabriqué dans le cadre de la fabrication du dispositif au moyen d'une empreinte en plastique d'une partie du corps (84) avec laquelle un contact doit être établi.
